# FASCICULE DE BREVET EUROPEEN

(11) **EP 3 074 367 B1**
(45) Date de publication et mention de la délivrance du brevet: **01.08.2018**
(21) Numéro de dépôt: 14805885.2
(22) Date de dépôt: 28.11.2014
(51) Int. Cl.: C07C 1/20, C07C 29/04

(54) **PROCEDE DE PRODUCTION DE 1,3-BUTADIÈNE À PARTIR D'UNE CHARGE COMPRENANT DE L'ÉTHANOL**
VERFAHREN ZUR HERSTELLUNG VON 1,3-BUTADIEN AUS EINEM ETHANOLHALTIGEN ROHSTOFF
PROCESS FOR PRODUCING 1,3-BUTADIENE FROM A FEEDSTOCK COMPRISING ETHANOL

(30) Priorité: 29.11.2013 FR 1361831
(43) Date de publication de la demande: 05.10.2016
(73) Titulaire: IFP Energies nouvelles, 92500 Rueil-Malmaison (FR); Compagnie Générale des Etablissements Michelin, 63000 Clermont-Ferrand (FR)
(72) Inventeur: DASTILLUNG, Rejane, 69005 Lyon (FR); CADRAN, Nicolas, 69600 Oullins (FR); FISCHER, Beatrice, 69005 Lyon (FR); JACQUIN, Marc, 69002 Lyon (FR); HUYGHE, Raphael, 69700 Saint Andeol Le Chateau (FR)
(86) Numéro de dépôt international: PCT/EP2014/076002
(87) Numéro de publication internationale: WO 2015/079040

(56) Documents cités:
- EP-A1- 1 052 234
- US-A- 2 374 433
- US-A- 2 474 874
- DATABASE CA [Online] CHEMICAL ABSTRACTS SERVICE, COLUMBUS, OHIO, US; LEBEDEV, S. V. ET AL: "The mechanism of the catalytic conversion of alcohols into biethylene hydrocarbons", retrieved from STN Database accession no. 1935:33471

## Description

### Domaine de l'invention

L'invention concerne le domaine de la production d'hydrocarbures insaturés, en particulier la production de dioléfines, comme par exemple le 1,3-butadiène, ce dernier pouvant être utilisé comme monomère de polymérisation, ou transformé en un autre intermédiaire chimique, comme par exemple la 1,6-hexanediamine.

### État de la technique antérieure

La production du 1,3-butadiène à partir d'éthanol est bien connue de l'homme du métier. Deux procédés ont été industrialisés à grande échelle :
- le procédé "S.K.", dans lequel le 1,3-butadiène est produit à partir d'éthanol en une étape réactionnelle, avec un unique catalyseur;
- le procédé "Carbide", dans lequel l'éthanol est converti en acétaldéhyde dans une première étape réactionnelle sur un premier catalyseur consistant en un mélange d'oxyde de chrome et d'oxyde de cuivre, suivie d'une deuxième étape réactionnelle sur un second catalyseur au cours de laquelle le mélange éthanol-acétaldéhyde est converti en 1,3-butadiène (voir par exemple US 2,439,587).

Le catalyseur utilisé dans le procédé "S.K." est déshydrogénant, et produit donc une quantité importante d'acétaldéhyde lorsqu'il est alimenté en éthanol. Dès les années 40, l'acétaldéhyde produit a été recyclé vers l'unité catalytique, permettant une amélioration significative des rendements. Par le biais de ce recyclage, l'unité catalytique du procédé "S.K." recevait donc un mélange éthanol-acétaldéhyde.

Le bilan global de la réaction de conversion de l'éthanol en 1,3-butadiène s'écrit comme suit :

2 CH₃CH₂OH ↔ CH₂=CHCH=CH₂ + H₂ + 2H₂O

Par soucis de simplification, on parlera donc par la suite de "charge riche en éthanol", ce terme désignant le mélange éthanol-acétaldéhyde comprenant plus de 50% poids d'éthanol envoyé vers l'unique catalyseur du procédé "S. K." ou vers le second catalyseur du procédé "Carbide".

Quelque soit le procédé (une ou deux étapes), la sélectivité de la transformation de la charge riche en éthanol en 1,3-butadiène est faible, ladite transformation conduisant à la formation de nombreux sous-produits. Par sous-produit, on entend les constituants formés autres que ceux produits ou consommés par la réaction principale (c'est à dire autre que les 1,3-butadiène, H₂, H₂O produits et éthanol et acétaldéhyde consommés). Toussaint et al., Industrial and Engineering Chemistry Vol. 39, No. 2, p. 120-125 1947 donnent à titre d'illustration la répartition des sous-produits formés sur une unité industrielle "Carbide" : diéthyle éther (23% poids des sous-produits), éthylène (11% poids des sous-produits), hexènes et hexadiènes (11% poids des sous-produits). Néanmoins, la répartition des sous-produits peut fortement évoluer suivant les conditions opératoires et la nature du catalyseur mis en oeuvre pour la transformation de la charge riche en éthanol en 1,3-butadiène.

Par ailleurs, la conversion de la charge riche en éthanol en 1,3-butadiène est faible. Les réactifs non convertis doivent donc être séparés des produits avant d'être recyclés. À titre d'illustration, le brevet US 2,393,381 décrit le recyclage de l'éthanol et de l'acétaldéhyde non convertis dans la seconde section catalytique du procédé "Carbide". Le brevet US 2,393,381 décrit aussi l'élimination des sous-produits gazeux et liquides générés par le procédé catalytique.

De nombreuses améliorations ont été apportées aux procédés de base, afin de valoriser certains sous-produits de ce type de procédé. En effet, les sous-produits peuvent être extraits par divers procédés de séparation puis :
- soit recyclés vers l'unité catalytique produisant du 1,3-butadiène à partir de la charge riche en éthanol.
- soit envoyés vers une unité catalytique dédiée pour être transformés en autres composés.

Ainsi, dans le brevet US 2,439,587 concernant le procédé "Carbide", l'acétate d'éthyle - impureté minoritaire du procédé - est valorisé par recyclage vers l'unité catalytique produisant du 1,3-butadiène. L'acétate d'éthyle peut être formé par différents mécanismes au sein de l'unité catalytique, mais peut également produire du 1,3-butadiène. En effet, dans les conditions réunies au sein de l'unité catalytique, l'acétate d'éthyle peut se substituer à l'éthanol pour produire du 1,3-butadiène et de l'acide acétique.

Un autre exemple bien connu de l'homme du métier est le cas du diéthyle éther (DEE), un sous-produit majoritaire du procédé. Dans le procédé "S. K." le DEE est renvoyé vers la section catalytique avec la charge riche en éthanol. Le recyclage du DEE vers l'unité catalytique permet d'améliorer le rendement en 1,3-butadiène, et entraine aussi une augmentation de la quantité d'éthylène produit. En effet, dans les conditions réunies au sein de l'unité catalytique, le DEE peut se substituer à l'éthanol pour produire du 1,3-butadiène et de l'éthylène.

En revanche, dans le procédé "Carbide", le recyclage du DEE vers l'unité catalytique n'entrainait pas d'augmentation du rendement en 1,3-butadiène. Cette différence de comportement peut s'expliquer aussi bien par la nature du catalyseur que part les niveaux thermiques, qui sont différents pour les deux procédés. Au sein du procédé "Carbide" (brevet US 2,474,874), le DEE est donc séparé, puis envoyé vers une unité catalytique dédiée afin d'être transformé en un autre composé valorisable, par exemple de l'éthylène. En effet, l'éthylène peut être utilisé comme matière première pour la synthèse de styrène, qui peut lui même être utilisé comme matière première pour la production de copolymère butadiène - styrène.

Un autre exemple de valorisation évoqué dans le brevet US 2,474,874 est la formation d'éthanol par hydratation du DEE. L'éthanol produit peut être recyclé sur site vers le procédé de production de 1,3-butadiène, de manière à augmenter le rendement global.

US 2,374,433 décrit un procédé de production de 1,3-butadiène à partir d'éthanol. Il est mentionné dans ce document qu'on peut recycler l'éthylène obtenu comme produit secondaire et l'ajouter à l'éthanol avant de commencer la réaction.

L'hydratation de l'éthylène en éthanol correspond à la réaction bilan suivante :

CH₂=CH₂ + H₂O → CH₃CH₂OH

Les procédés d'hydratation de l'éthylène en éthanol sont bien connus de l'homme du métier et se divisent en deux grandes catégories : hydratation directe et indirecte (Weissermel et Arpe, Industrial Organic Chemistry 4ème édition, Wiley-VCH 2003).

L'hydratation indirecte se caractérise par la formation d'intermédiaires réactionnels formés par l'addition d'un acide sur l'oléfine, suivie d'une hydrolyse en alcool. Ce type de procédé peut être réalisé en présence d'acide sulfurique concentré qui, en présence de gaz contenant de l'éthylène, forme des mono- et diéthylsulfates, lesquels se décomposent en éthanol après ajout d'eau dans le milieu. Les avantages de ce type de procédé sont les faibles températures de réactions (50-150°C) et un niveau de conversion par passe élevé. Le principal désavantage est l'utilisation d'acide concentré, qui pose des problèmes de corrosion ainsi que des coûts opératoires importants pour la reconcentration / réutilisation de l'acide. L'hydratation indirecte est réalisée en milieu diphasique gaz/liquide.

L'hydratation directe se caractérise par la formation en une seule étape réactionnelle de l'alcool. Pour l'hydratation de l'éthylène, la réaction se fait en phase gaz. Les températures de réaction sont plus élevées (200-400°C) et les conversions par passe faible (<10%) ce qui nécessite un recyclage important. Ce procédé utilise des catalyseurs hétérogènes à base d'acides inorganiques comme l'acide phosphorique déposés par exemple sur des supports siliciques ou à base de silice (kieselguhr..) ce qui permet d'éliminer les problèmes liés à l'utilisation d'un acide concentré nécessaire au procédé d'hydratation indirect. Les inconvénients de ce type de procédé sont sa faible conversion par passe et son besoin de travailler avec de l'éthylène de haute pureté (notamment pour éviter l'accumulation d'inertes lors du recycle).

Les procédés d'hydratation de l'éthylène, direct ou indirect, nécessitent de travailler avec un éthylène très pur. En effet, la présence d'impuretés est problématique pour le bon fonctionnement de ces procédés. On peut illustrer ce propos avec deux impuretés classiques de la coupe de distillation "C₂" que sont l'éthane et l'acétylène. Au sein d'un procédé d'hydratation direct, l'éthane s'accumule car non converti. Au sein d'un procédé d'hydratation direct ou indirect, l'acétylène est transformé en acétaldéhyde, qu'il faut gérer et éliminer au sein du procédé.

Par ailleurs, même avec un éthylène de haute pureté, l'éthanol produit doit être purifié. En effet, des réactions secondaires peuvent se produire et générer des sous-produits comme par exemple l'acétaldéhyde ou le diéthyle éther.

Or, il a été découvert qu'il était possible d'associer judicieusement une étape de conversion de l'éthanol en 1,3-butadiène et une étape d'hydratation de l'éthylène en éthanol de manière à accroître le rendement global en 1,3-butadiène, et ce malgré une baisse de la conversion et de la sélectivité dans l'étape d'hydratation due à l'utilisation d'un éthylène moins pur qu'enseigné dans l'art antérieur.

Ladite association, par le jeu des recyclages et des synergies, permet de valoriser des sous-produits communs, alors que ces sous-produits (par exemple le DEE et l'acétaldéhyde) doivent être éliminés lorsque ces étapes sont opérées indépendamment l'une de l'autre.

### Résumé et intérêt de l'invention

Un aspect de l'invention est de valoriser le sous-produit éthylène produit lors de la transformation de la charge riche en éthanol en 1,3-butadiène, afin de maximiser le rendement en 1,3-butadiène.

Le sous-produit éthylène produit avec le 1,3-butadiène est concentré dans un flux gazeux qui est hydraté dans une section catalytique dédiée afin de produire majoritairement de l'éthanol. L'effluent ainsi obtenu est renvoyé au sein de l'étape de conversion en 1,3-butadiène via une étape de séparation à même de séparer les différents constituants, par exemple l'éthanol, l'eau, le DEE, l'acétaldéhyde et l'éthylène. Ainsi, l'impureté éthylène est valorisée, ce qui permet de maximiser le rendement global en 1,3-butadiène.

Cette invention est particulièrement adaptée quand, du fait par exemple des conditions opératoires, ou du catalyseur mis en oeuvre, ou du recyclage du diéthyle éther au sein de l'unité de transformation de la charge riche en éthanol en 1,3-butadiène, la quantité d'éthylène produite est importante.

Un autre aspect de l'invention est d'alimenter en totalité ou partiellement l'unité d'hydratation de l'éthylène avec une autre source d'éthylène, afin d'assurer une plus grande flexibilité à l'opérateur de l'unité de production de 1,3-butadiène en fonction de l'évolution du coût des matières premières, des prix marché des produits et des prix des énergies. Cette autre source d'éthylène peut par exemple être une unité de vapocraquage d'hydrocarbures fossiles ou une unité de type craquage catalytique en lit fluidisé, ou Fluid Catalytic Cracking dans la langue de Shakespeare.

Enfin, un autre aspect de l'invention est d'utiliser, en totalité ou partiellement, l'eau coproduite avec le 1,3-butadiène pour hydrater l'éthylène, que celui-ci soit un sous-produit généré au sein du procédé ou provienne d'une autre source.

La demanderesse a découvert qu'il était possible d'alimenter l'unité d'hydratation avec de l'éthylène moins pure que ce qui est fait habituellement dans l'art antérieur, en obtenant, malgré une hydratation ne fonctionnant pas de manière optimale, une amélioration des performances globales du procédé de production de 1,3-butadiène.

L'invention impacte à la baisse le niveau des investissements et les consommations en utilités par rapport à l'art antérieur (eau, vapeur, électricité). En effet :
- L'éthanol produit par l'étape **C** d'hydratation de l'éthylène n'a pas besoin d'être purifié à 94.5% massique, comme communément effectué dans l'art antérieur, car il est recyclé en tant que charge dans l'étape **A** de conversion, où la contrainte de pureté est plus faible.
- Les étapes de séparation et de traitement des effluents des étapes **A** de conversion et **C** d'hydratation sont mutualisées. Il n'est donc plus besoin d'un train dédié de purification de l'effluent d'hydratation.
- L'acétaldéhyde et le DEE produits par la réaction d'hydratation de l'éthylène sont, dans l'état de l'art, séparés de l'éthanol et le plus souvent torchés, c'est à dire brulés à perte. Ces produits étant également des réactifs et sous-produits de l'étape **A** de conversion, il n'est plus nécessaire d'avoir des colonnes dédiées pour les soutirer de l'éthanol produit à partir de l'éthylène. L'intégration de l'étape **A** de conversion et de l'étape **C** d'hydratation de l'éthylène permet de valoriser l'acétaldéhyde et le DEE.
- L'éthylène produit par l'étape **A** de conversion et envoyé vers l'unité d'hydratation n'a pas besoin d'être purifié à la hauteur de 99.9% pds, ce qui limite les pertes produits et les niveaux d'investissement.
- L'eau produite par l'étape **A** de conversion est utilisée totalement ou partiellement dans l'étape **C** d'hydratation, ce qui limite les rejets de l'unité, et donc la taille et la consommation en utilités de l'unité de traitement des eaux en aval.

### DESCRIPTION DE L'INVENTION

L'invention concerne un procédé de production de 1,3-butadiène à partir d'une charge riche en éthanol, c'est-à-dire dans laquelle l'éthanol représente plus de 50% du poids total de ladite charge, comprenant au moins :
A) Une étape de conversion d'au moins ladite charge riche en éthanol et de l'effluent éthanol issu de l'étape B de séparation en un effluent de conversion comprenant majoritairement du 1,3-butadiène, de l'eau et de l'éthylène, et en un effluent hydrogène, opérant à une pression comprise entre 0,1 et 1,0 MPa, à une température comprise entre 300 et 500°C en présence d'au moins un catalyseur;
B) Une étape de séparation d'au moins ledit effluent de conversion issu de A et l'effluent d'hydratation issu de C en au moins un effluent éthanol, un effluent butadiène, un effluent eau et un effluent éthylène;
C) Une étape d'hydratation d'éthylène alimentée au moins par ledit effluent éthylène et/ou ledit effluent eau issus de l'étape B de séparation, afin de produire un effluent d'hydratation comprenant de l'éthanol, ledit effluent d'hydratation étant ensuite recyclé vers l'étape B de séparation.

### Charge riche en éthanol

Selon l'invention, la charge envoyée vers l'unité de production de 1,3-butadiène est une charge riche en éthanol. Par charge riche en éthanol, on entend une charge dans laquelle l'éthanol représente plus de 50% du poids total de ladite charge.

Ladite charge riche en éthanol peut avantageusement comprendre de l'acétaldéhyde. Lorsque ladite charge riche en éthanol comprend de l'acétaldéhyde, le rapport massique éthanol sur acétaldéhyde est préférentiellement compris entre 2:1 et 4:1, de manière préférée entre 2,5:1 et 3,5:1 et de manière très préférée de 3:1.

Ladite charge riche en éthanol peut également comprendre des impuretés, telles que par exemple des éthers (diéthyle éther, vinyléthyle éther, methyléthyle éther, butyléthyle éther,...), des esters (par exemple l'éthyle acétate), des acétals (par exemple l'éthyle acétal), des aldéhydes autres que l'acétaldéhyde et des cétones (par exemple le crotonaldéhyde, le butyraldéhyde et l'acétone), des alcools (par exemple le butanol, l'héxanol,...), et des hydrocarbures saturés ou insaturés. La teneur de ces impuretés est comprise entre 0 % et 20% du poids total de la charge, de préférence entre 0 et 10% poids.

Enfin, ladite charge riche en éthanol peut également comprendre de l'eau. La teneur en eau est avantageusement comprise entre 0 et 30% du poids total de la charge. De manière préférée, la teneur en eau est comprise entre 4 et 15% pds.

### Étape A de conversion de la charge riche en éthanol en 1,3-butadiène

Conformément à l'invention, ladite charge riche en éthanol alimente une étape **A** de conversion.

Selon un mode préféré, ladite étape **A** de conversion est opérée en une étape réactionnelle (procédé « S.K. »). Dans ce mode, ladite charge riche en éthanol est mélangée à l'effluent éthanol issu de l'étape B de séparation avant d'alimenter ladite étape **A** de conversion.

Selon un autre mode préféré, ladite étape **A** est opérée en deux étapes réactionnelles, la première étape permettant de convertir l'éthanol en acétaldéhyde en présence d'un catalyseur consistant en un mélange d'oxyde de chrome et d'oxyde de cuivre, ou de tout autre catalyseur adapté. Le rapport massique éthanol sur acétaldéhyde dans l'effluent de ladite première étape réactionnelle est préférentiellement compris entre 2:1 et 4:1, de manière préférée entre 2,5:1 et 3,5:1 et de manière très préférée de 3:1. Dans ce mode, ladite charge riche en éthanol alimente la première étape réactionnelle et l'effluent éthanol issu de l'étape **B** de séparation alimente la deuxième étape réactionnelle, en mélange avec ledit effluent de la première étape réactionnelle.

Ladite charge riche en éthanol et l'effluent éthanol issu de l'étape **B** subissent, dans une étape **A** de conversion, une transformation chimique de manière à produire un effluent de conversion et un effluent hydrogène. Ladite étape **A** convertit totalement ou partiellement la charge riche en éthanol et l'effluent éthanol issu de l'étape **B** au moins en butadiène, éthylène, hydrogène, eau, et acétaldéhyde, dont majoritairement du 1,3-butadiène. Par majoritairement, on entend que plus de 50%pds des produits formés (hors production d'eau, d'hydrogène et d'acétaldéhyde) à l'issue de l'étape **A** sont du 1,3-butadiène, de préférence plus de 60%pds. Néanmoins, la conversion de la charge riche en éthanol et de l'effluent éthanol issu de l'étape **B** pouvant être partielle, le 1,3-butadiène peut représenter moins de 50% pds de l'effluent de conversion (hors eau, hydrogène et acétaldéhyde) du fait, par exemple, de la présence d'éthanol non converti. Ledit effluent de conversion comprend entre 1 et 8% poids d'éthylène (hors eau, éthanol, hydrogène et acétaldéhyde).

L'étape **A** est opérée à une pression comprise entre 0,1 et 1,0 MPa, de préférence entre 0,1 et 0,5 MPa, de manière préférée entre 0,1 et 0,3 MPa. L'étape **A** est opérée à une température comprise entre 300 et 500°C.

Dans le cas où l'étape **A** est opérée en une étape réactionnelle,elle est opérée en présence d'un catalyseur de type aluminate de zinc, ou MgO-SiO₂ dopé au Chrome, comme celui utilisé dans le procédé "S.K." (voir par exemple Bhattacharyya, Ganguly Journal of Applied Chemistry Volume 12, Issue 3, pages 97-110, March 1962). L'étape **A** est alors opérée de manière préférée à une température comprise entre 380 et 430°C.

Dans le cas où l'étape **A** est opérée en deux étapes réactionnelle la deuxième étape réactionnelle de ladite étape **A** est opérée en présence d'un catalyseur de type silice avec un oxyde de tantale, de zirconium ou de colombium, préférentiellement avec 2% d'oxyde de Tantale, comme celui utilisé dans le procédé "Carbide" (voir par exemple Corson, Jones, Welling, Hincbley, Stahly, Ind. Eng Chem. 1950, 42, 2, 359-373). La deuxième étape réactionnelle de l'étape **A** est alors opérée de manière préférée à une température comprise entre 320 et 370°C. La première étape réactionnelle de ladite étape **A** est opérée conformément à l'art antérieur, à une température comprise entre 200 et 300°C.

Un effluent hydrogène, comprenant majoritairement de l'hydrogène, est séparé à l'issue de la réaction par des moyens connus de l'Homme du métier (séparateur gaz-liquide par exemple).

Lorsque l'étape **A** de conversion est mise en oeuvre avec deux étapes réactionnelles (conversion selon le procédé « Carbide »), l'hydrogène est majoritairement produit dans la première étape réactionnelle du procédé. Il est alors séparé entre les deux étapes réactionnelles.

### Étape B de séparation

Conformément à l'invention, l'effluent de conversion issu de l'étape **A** ainsi que l'effluent d'hydratation issu de l'étape **C** alimentent une étape **B** de séparation de manière à produire au moins un effluent éthanol, un effluent butadiène, un effluent eau, et un effluent éthylène.

Ladite étape **B** permet de séparer le butadiène, produit principal du procédé selon l'invention, ainsi que de produire un effluent éthanol apte à être recyclé dans l'étape **A** de conversion.

Par effluent éthanol, on entend un effluent comprenant plus de 50% pds d'éthanol. Cet effluent peut contenir jusqu'à 30% pds d'acétaldéhyde. Cet effluent peut également comprendre des impuretés, telles que par exemple des éthers (par exemple le diéthyle éther, le vinyléthyle éther, le methyléthyle éther, le butyléthyle éther,...), des esters (par exemple l'éthyle acétate), des acétals (par exemple l'éthyle acétal), des aldéhydes et des cétones (par exemple le crotonaldéhyde, le butyraldéhyde et l'acétone), des alcools (par exemple le butanol, l'héxanol,...), et des hydrocarbures saturés ou insaturés. La teneur totale de ces impuretés est comprise entre 0 % et 20% pds, de préférence entre 0 et 10% pds. Cet effluent peut également contenir de l'eau. La teneur en eau peut être comprise entre 0 et 30% pds. De manière préférée, la teneur en eau est comprise entre 4 et 15% pds. L'acétaldéhyde peut avantageusement être ultérieurement séparé de l'effluent éthanol.

Ledit effluent éthanol est recyclé dans l'étape **A** en mélange avec ladite charge riche en éthanol.

Par effluent butadiène, on entend un effluent comprenant plus de 80% pds, de préférence plus de 90% pds, et de manière préférée plus de 99% pds de 1,3-butadiène.

Par effluent eau, on entend un effluent comprenant plus de 90% pds d'eau, de préférence plus de 99% pds d'eau. Cet effluent peut notamment contenir des impuretés très polaires et moins volatiles que l'eau, notamment de l'acide acétique.

Par effluent éthylène, on entend un effluent gazeux à une température inférieure à 50°C et une pression inférieure à 0,8 MPa, comprenant au moins 50% poids d'éthylène. Il peut également comprendre des traces d'hydrogène, du monoxyde de carbone, du dioxyde de carbone, du propylène, de l'acétylène, des alcanes légers (par exemple méthane, éthane, propane), du DEE, pouvant être formés par des réactions secondaires.

L'étape **B** de séparation produit également avantageusement un effluent gaz lourds, c'est-à-dire un effluent gazeux à une température inférieure à 50°C et une pression inférieure à 0,8 MPa comprenant les constituants autres que ceux compris dans l'effluent éthylène.

L'étape **B** de séparation produit également avantageusement un effluent huiles, c'est à dire un effluent comprenant un mélange de composés d'hydrocarbures saturés et insaturés, et de composés oxygénés : esters, éthers, acétals, aldéhydes, cétones, alcools pouvant être saturés ou insaturés. Cet effluent se caractérise par le fait de n'être composé que de carbone, d'oxygène et d'hydrogène, et d'avoir une température d'ébullition pouvant aller de 20°C à 500°C. Il peut être utilisé comme combustible pour la génération des utilités (chaleur, électricité) nécessaires au bon fonctionnement du procédé selon l'invention, ou bien être traité pour des opérations de séparations pour en extraire des produits valorisables.

L'étape **B** de séparation est réalisée par un ensemble d'opérations unitaires bien connues de l'homme du métier, par exemple et de manière non limitative par distillation, distillation cryogénique, lavage par solvant (par exemple eau ou éthanol), distillation extractive, extraction liquide-liquide, passage sur tamis, séparation membranaire. Ainsi, l'étape **B** de séparation est de préférence choisie parmi les étapes de distillation, distillation cryogénique, lavage par solvant, distillation extractive, extraction liquide-liquide, passage sur tamis, séparation membranaire et les combinaisons de ces étapes entre-elles.

De manière non limitative, un exemple d'enchaînement de ces opérations unitaires peut être le suivant. L'effluent de conversion issu de l'étape **A** est refroidit et envoyé dans une colonne de lavage à l'éthanol : un effluent éthylène, comprenant de l'éthylène, de l'éthanol et éventuellement des traces d'hydrogène, est évacué en tête de la colonne de lavage. L'effluent liquide obtenu en fond de la colonne de lavage est envoyé vers une colonne à distiller, afin d'obtenir un effluent riche en butadiène en tête de la colonne à distiller. Cet effluent riche en butadiène est ensuite lavé à l'eau pour éliminer les composés polaires volatiles. Enfin l'effluent riche butadiène ayant été lavé à l'eau est envoyé vers une unité de distillation extractive utilisant un solvant polaire (par exemple le DMF, la NMP ou l'ACN) afin d'éliminer les butènes et éventuellement d'autres impuretés de manière à produire un effluent butadiène. Le fond la colonne à distiller et des colonnes de lavage sont envoyés vers un train de distillation qui permet de produire un effluent eau, un effluent éthanol, et un effluent huiles. L'effluent éthanol peut être recyclé vers l'étape de conversion A, en mélange avec la charge riche en éthanol.

### Étape C d'hydratation

Conformément à l'invention, au moins une partie de l'effluent éthylène et/ou au moins une partie de l'effluent eau issus de l'étape **B** de séparation alimentent une étape **C** d'hydratation de manière à produire un effluent d'hydratation.

Ladite étape **C** est avantageusement alimentée par une autre source externe d'éthylène. Ladite autre source externe d'éthylène peut être, par exemple, un vapocraqueur ou une unité de type FCC. Ladite étape **C** peut également avantageusement être alimentée par une source externe d'eau.

La pureté du flux d'éthylène alimentant l'étape **C** d'hydratation est donc variable selon la source d'éthylène utilisée. La pureté de l'éthylène est comprise entre 50 et 100%, de manière préférée entre 65 et 99,9% poids, et de manière très préférée entre 65 et 75% poids.
Dans un mode préféré, ladite étape **C** d'hydratation est une hydratation indirecte. Dans une première étape, l'éthylène réagit en présence d'acide sulfurique concentré à une température de réaction comprise entre 50 et 150°C, en milieu diphasique gaz/liquide. Dans une seconde étape, les produits formés dans la première étape sont hydrolysés pour former de majoritairement de l'éthanol à une température comprise entre 70 et 100°C. Enfin, l'acide sulfurique est reconcentré pour être réutilisé.

Dans un autre mode préféré, ladite étape **C** d'hydratation est une hydratation directe, réalisée en phase gaz, à une température de réaction comprise entre 200 et 400°C, en présence d'un catalyseur hétérogène à base d'acides inorganiques comme l'acide phosphorique déposés par exemple sur des supports siliciques ou à base de silice (kieselguhr..).

Conformément à l'invention, ledit effluent d'hydratation est recyclé vers l'étape **B** de séparation. Il peut être soit mélangé directement avec l'effluent de conversion issu de l'étape **A** de conversion, soit traité par une opération unitaire dédiée de l'étape **B** de séparation. Ce recyclage permet, *in fine,* le recyclage de l'éthanol vers l'étape **A** de conversion.

Ledit effluent d'hydratation comprend, outre de l'éthanol, du DEE produit selon la réaction suivante :

2 CH₂CH₂ + H₂O ↔ CH₃CH₂OCH₂CH₃

Du fait de la faible conversion (moins de 15% de l'éthylène converti, préférentiellement moins de 10%), l'effluent d'hydratation comprend d'une part des sous-produits gazeux n'ayant pas totalement réagit dans l'unité catalytique **C** (propylène acétylène, DEE), et d'autre part des sous-produits gazeux ne réagissant pas (monoxyde de carbone, dioxyde de carbone, méthane, éthane, propane) qui sont envoyés dans l'étape **B** de séparation de manière à maintenir constante la concentration en sous-produits gazeux ne réagissant pas dans l'alimentation de l'étape **C.**

Dans un mode préféré, la totalité de l'éthylène alimentant l'unité catalytique **C** est produit par l'étape **A** de conversion, après passage dans l'étape **B** de séparation. C'est à dire qu'il n'y a pas d'apport par une source externe d'éthylène.

Dans ce mode, la quantité d'eau produite dans l'étape **A** est suffisante pour permettre d'hydrater l'éthylène produit dans l'étape **A.** Par conséquent, la totalité de l'eau alimentant l'étape **C** d'hydratation peut provenir de l'étape **A** de conversion, après séparation dans l'étape **B** de séparation.

L'étape **C** d'hydratation est également avantageusement alimentée par de l'éthylène provenant d'une source externe.

Si la quantité d'eau produite dans l'étape **A** n'est pas suffisante pour hydrater l'éthylène alimentant l'étape **C** d'hydratation, alors une source externe supplémentaire d'alimentation en eau de l'étape **C** est également utilisée. La part d'eau provenant de ladite source externe représente moins de 20% poids, préférentiellement moins de 10% poids de l'eau alimentant l'étape **C** d'hydratation.

Lorsque le procédé selon l'invention est mis en oeuvre dans un site comportant d'autres procédés, les flux dudit site comprenant de l'acétylène ou du DEE peuvent avantageusement être traités dans l'étape **C** d'hydratation du procédé selon l'invention.

### Description des figures

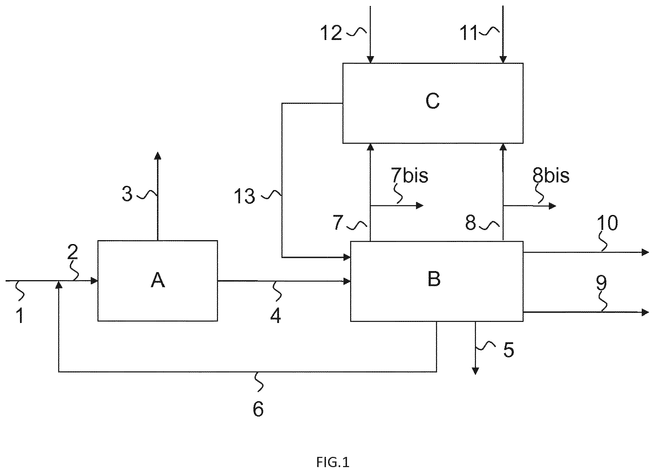

La figure 1 décrit schématiquement le procédé de production de 1,3-butadiène à partir d'une charge riche en éthanol selon l'invention.

La charge riche en éthanol **1** est mélangée à l'effluent éthanol **6** de manière à former une charge de conversion **2.** Ladite charge de conversion **2** est envoyée dans l'étape **A** de conversion de manière à produire un effluent de conversion **4** et un effluent hydrogène **3.**

L'effluent de conversion **4** issu de l'étape **A** et l'effluent d'hydratation **13** issu de l'étape **C** alimentent l'étape **B** de séparation dans laquelle sont séparés un effluent éthanol **6,** un effluent butadiène **5,** un effluent eau **7,** un effluent éthylène **8,** un effluent gaz lourds **9,** et un effluent huiles **10.**

Une fraction **7bis** de l'effluent eau **7** est purgée. Une fraction **8bis** de l'effluent éthylène **8** est purgée. Une partie de l'effluent eau **7** et une partie de l'effluent éthylène **8** alimentent l'étape **C** d'hydratation. Une source externe d'éthylène **11** ainsi qu'une source externe d'eau **12** alimentent également ladite étape **C.**

L'étape **C** produit un effluent d'hydratation **13,** lequel alimente l'étape **B** de séparation.

Les exemples suivants illustrent l'invention sans en limiter la portée.

### Exemples

Dans les exemples suivant, les performances des procédés sont évaluées sur la base du rendement global en 1,3-butadiène défini comme suit : débit massique de 1,3-butadiène dans l'effluent butadiène divisé par le débit massique d'éthanol dans la charge riche en éthanol.

### Exemple 1 - non conforme

*L'exemple 1 illustre le fonctionnement du procédé Lebedev selon l'art antérieur. Après une étape de conversion, l'éthanol, ainsi que l'acétaldéhyde non convertis sont séparés et recyclés en amont de ladite étape de conversion.*

Une charge riche en éthanol constituée de 93,3%pds d'éthanol et de 6,7%pds d'eau alimente une l'étape **A** de conversion. L'éthanol et l'acétaldéhyde non convertis présents dans l'effluent de conversion sont séparés dans une étape **B** de séparation et recyclés en amont de l'étape **A.**

L'étape **B** de séparation est opérée de telle sorte que 99% de l'éthanol et 100% de l'acétaldéhyde compris dans l'effluent de conversion sont recyclés vers l'étape **A.**

Le rendement global en 1,3-butadiène du procédé est de 0,383.

### Exemple 2 - non conforme

*Cet exemple a pour base l'exemple 1. Après l'étape de conversion, on sépare en outre un effluent éthylène qui est hydraté dans un procédé d'hydratation tel que connu de l'Homme du métier de manière à produire un effluent d'hydratation comprenant de l'éthanol. Ledit effluent éthanol est ensuite recyclé en amont de l'étape de conversion (après séparation de l'éthylène et d'une partie de l'eau).*

L'étape **A** de conversion est alimentée par une charge riche en éthanol identique à celle de l'exemple 1, ainsi que par un effluent éthanol issu de l'étape **B** de séparation.

L'étape **B** de séparation est opérée de telle sorte que 99% de l'éthanol et 100% de l'acétaldéhyde compris dans l'effluent de conversion sont recyclés vers l'étape **A.**

Comme il est connu de l'Homme du métier, l'éthylène envoyé vers l'unité d'hydratation doit être très pur (Weissermel et Arpe, Industrial Organic Chemistry 4ème édition, Wiley-VCH 2003). La séparation et la purification de l'éthylène contenu dans ledit effluent de conversion en un effluent éthylène comprenant 99,9% pds d'éthylène induit 15% de perte d'éthylène. Ainsi, l'étape **B** de séparation permet de récupérer 85% de l'éthylène compris dans ledit effluent de conversion.

Ledit effluent éthylène, ainsi qu'un flux d'eau d'origine externe au procédé, sont convertis en éthanol dans un procédé d'hydratation tel que connu de l'Homme du métier. À l'issue de l'étape de réaction d'hydratation, l'éthanol est purifié dans une unité de séparation dédiée typique des procédés d'hydratation de l'art antérieur, qui produit un éthanol à 94,5% pds, et recyclé vers l'alimentation de l'étape **A** de conversion.

Le rendement global du procédé est de 0,395, soit 3,1% de plus que dans l'exemple 1.

### Exemple 3 - Conforme

*Dans cet exemple, conforme à l'invention, l'effluent de conversion est traité dans une étape **B** de séparation de manière à produire au moins un effluent butadiène, un effluent éthanol, un effluent éthylène et un effluent eau. La spécification de pureté sur l'effluent éthylène est moindre, et un flux d'eau interne au procédé est utilisé pour l'étape d'hydratation.*

L'étape **A** de conversion est alimentée par une charge riche en éthanol identique à celle de l'exemple 1, ainsi que par un effluent éthanol issu de l'étape **B** de séparation.

L'étape **B** de séparation, alimentée par l'effluent de conversion issu de ladite étape **A,** ainsi que par l'effluent d'hydratation issu de ladite étape **C,** permet de produire au moins un effluent butadiène, un effluent éthanol, un effluent éthylène et un effluent eau. Elle est opérée de telle sorte que 99% de l'éthanol et 100% de l'acétaldéhyde compris dans l'alimentation de ladite étape **B** sont recyclés vers l'étape **A.** 99% de l'éthylène compris dans ledit effluent de conversion est séparé dans ledit effluent éthylène. La pureté de l'éthylène dans cet effluent est de 73% pds. Il est envoyé, ainsi que ledit effluent eau, vers une étape **C** d'hydratation

Le rendement global du procédé est de 0,397, soit 0,5% de mieux que l'exemple 2 et 3,7% de mieux que l'exemple 1.

En référence à l'exemple 2, la performance globale du procédé a pu être maintenue, voire améliorée de 0,5% alors que la pureté de l'éthylène envoyé vers l'étape d'hydratation est bien inférieure (73% pds au lieu de 99,9% pds). La séparation de l'éthylène contenu dans l'effluent de conversion issu de l'étape **A** est donc, du fait d'une contrainte de pureté moins sévère, facilitée dans l'exemple selon l'invention, ce qui permet une meilleure récupération dudit éthylène (99% au lieu de 85%). Cette pureté plus faible conduit également à une séparation moins énergivore. La mise en commun du traitement de l'effluent de conversion issu de l'étape **A** et de l'effluent d'hydratation issu de l'étape **C** permet de réduire de 40% le nombre d'équipements nécessaires.

Le procédé selon l'invention permet donc une meilleure valorisation de l'éthylène co-produit dans l'étape **A** de conversion, puisque l'on valorise 16,4% d'éthylène en plus par rapport à l'exemple 2 (99% / 85%).

L'hydratation d'un effluent éthylène de faible pureté, contrairement aux usages connus de l'art antérieur, n'a, de manière surprenante, pas pour effet d'impacter le rendement global en 1,3-butadiène du procédé. En effet, même si l'effluent eau et l'effluent éthylène qui alimentent ladite étape d'hydratation comprennent des impuretés, comme par exemple l'acide acétique, l'acétaldéhyde, l'acétylène, le propylène et le diéthyle éther, la mise en commun de l'étape de séparation **B** apte à séparer ces composés et le recyclage vers l'étape de conversion **A** qui converti certaines de ces impuretés compensent les dégradations de performances de l'étape **C** d'hydratation liées à l'utilisation de charge (effluent éthylène et eau) moins purs que celles communément utilisées dans l'art antérieur.

## Revendications

1. Procédé de production de 1,3-butadiène à partir d'une charge riche en éthanol, c'est-à-dire dans laquelle l'éthanol représente plus de 50% du poids total de ladite charge, comprenant au moins :
A) Une étape de conversion d'au moins ladite charge riche en éthanol et de l'effluent éthanol issu de l'étape B de séparation en un effluent de conversion comprenant majoritairement du 1,3-butadiène, de l'eau et de l'éthylène, et en un effluent hydrogène, opérant à une pression comprise entre 0,1 et 1,0 MPa, à une température comprise entre 300 et 500°C en présence d'au moins un catalyseur;
B) Une étape de séparation d'au moins ledit effluent de conversion issu de A et l'effluent d'hydratation issu de C en au moins un effluent éthanol, un effluent butadiène, un effluent eau et un effluent éthylène;
C) Une étape d'hydratation d'éthylène alimentée au moins par ledit effluent éthylène et/ou ledit effluent eau issus de l'étape B de séparation, afin de produire un effluent d'hydratation comprenant de l'éthanol, ledit effluent d'hydratation étant ensuite recyclé vers l'étape B de séparation.

2. Procédé selon la revendication 1 dans lequel ladite étape **A** est opérée à une pression comprise entre 0,1 et 0,5 MPa.

3. Procédé selon l'une des revendications 1 à 2 dans lequel ladite étape **A** de conversion est opérée en une étape réactionnelle et dans lequel ladite charge riche en éthanol est mélangée à l'effluent éthanol issu de l'étape **B** de séparation avant d'alimenter ladite étape **A** de conversion.

4. Procédé selon la revendication 3 dans lequel ladite étape **A** est opérée en présence d'un catalyseur de type aluminate de zinc, ou MgO-SiO₂ dopé au Chrome à une température comprise entre 380 et 430°C.

5. Procédé selon l'une des revendications 1 à 2 dans lequel ladite étape **A** est opérée en deux étapes réactionnelles, la première étape permettant de convertir l'éthanol en acétaldéhyde en présence d'un catalyseur consistant en un mélange d'oxyde de chrome et d'oxyde de cuivre, ou de tout autre catalyseur adapté, le rapport massique éthanol sur acétaldéhyde dans l'effluent de ladite première étape réactionnelle étant préférentiellement compris entre 2:1 et 4:1, ladite charge riche en éthanol alimentant ladite première étape réactionnelle et ledit effluent éthanol issu de ladite étape **B** de séparation alimentant ladite deuxième étape réactionnelle, en mélange avec ledit effluent de ladite première étape réactionnelle.

6. Procédé selon la revendication 5 dans lequel la deuxième étape réactionnelle de ladite étape **A** est opérée en présence d'un catalyseur de type silice avec un oxyde de tantale, de zirconium ou de colombium, la deuxième étape réactionnelle de l'étape **A** étant opérée à une température comprise entre 320 et 370°C, la première étape réactionnelle de ladite étape **A** étant opérée à une température comprise entre 200 et 300°C.

7. Procédé selon l'une des revendications 1 à 6 dans lequel l'étape **B** de séparation est choisie parmi les étapes de distillation, distillation cryogénique, lavage par solvant, distillation extractive, extraction liquide-liquide, passage sur tamis, séparation membranaire et les combinaisons de ces étapes entre-elles.

8. Procédé selon l'une des revendications 1 à 7 dans lequel ladite étape **C** est une hydratation indirecte, dans laquelle, dans une première étape réactionnelle, l'éthylène réagit en présence d'acide sulfurique concentré à une température de réaction comprise entre 50 et 150°C, en milieu diphasique gaz/liquide, et dans une seconde étape, les produits formés dans la première étape réactionnelle sont hydrolysés pour former de majoritairement de l'éthanol à une température comprise entre 70 et 100°C.

9. Procédé selon l'une des revendications 1 à 7 dans lequel ladite étape **C** est une hydratation directe opérant en phase gaz, à une température de réaction comprise entre 200 et 400°C, en présence d'un catalyseur hétérogène à base d'acides inorganiques comme l'acide phosphorique déposés sur des supports à base de silice.

10. Procédé selon l'une des revendications 1 à 9 dans lequel ladite charge riche en éthanol comprend de l'acétaldéhyde, le rapport massique éthanol sur acétaldéhyde étant compris entre 2:1 et 4:1.

11. Procédé selon l'une des revendications 1 à 10 dans lequel la pureté du flux d'éthylène alimentant l'étape **C** d'hydratation est comprise entre 65 et 99,9% poids.

12. Procédé selon l'une des revendications 1 à 10 dans lequel la pureté du flux d'éthylène alimentant l'étape **C** d'hydratation est comprise entre 65 et 75% poids.

## Patentansprüche

1. Verfahren zur Herstellung von 1,3-Butadien aus einem ethanolreichen Rohstoff, das heißt, wobei das Ethanol mehr als 50 % des Gesamtgewichts des Rohstoffs ausmacht, umfassend mindestens:
A) Einen Schritt zur Umwandlung mindestens des ethanolreichen Rohstoffs und des aus dem Trennschritt B stammenden Ethanolabstroms in einen Umwandlungsabstrom, umfassend vorwiegend 1,3-Butadien, Wasser und Ethylen, und einen Wasserstoffabstrom, der bei einem Druck zwischen 0,1 und 1,0 Mpa, bei einer Temperatur zwischen 300 und 500 °C, in Gegenwart von mindestens einem Katalysator durchgeführt wird;
B) Einen Schritt zum Abtrennen mindestens des aus A stammenden Umwandlungsabstroms und des aus C stammenden Hydrierungsabstroms in mindestens einen Ethanolabstrom, einen Butadienabstrom, einen Wasserabstrom und einen Ethylenabstrom;
C) Einen Schritt zur Etylenhydrierung, der mindestens durch den Ethylenabstrom und/oder den Wasserabstrom, die beide aus dem Trennschritt B stammen, gespeist wird, um einen Hydrierungsabstrom zu erzeugen, der Ethanol umfasst, wobei der Hydrierungsabstrom anschließend in den Trennschritt B zurückgeführt wird.

2. Verfahren nach Anspruch 1, wobei der Schritt A bei einem Druck zwischen 0,1 und 0,5 MPa durchgeführt wird.

3. Verfahren nach einem der Ansprüche 1 bis 2, wobei der Umwandlungsschritt A in einem Reaktionsschritt durchgeführt wird und wobei der ethanolreiche Rohstoff mit dem aus dem Trennschritt B stammenden Ethanolabstrom vor dem Einspeisen in den Umwandlungsschritt A gemischt wird.

4. Verfahren nach Anspruch 3, wobei der Schritt A in Gegenwart eines Zinkaluminat- oder mit Chrom dotierten MgO-SiO₂-Katalysators bei einer Temperatur im Bereich zwischen 380 und 430 °C arbeitet.

5. Verfahren nach einem der Ansprüche 1 bis 2, wobei der Schritt A in zwei Reaktionsschritten durchgeführt wird, wobei der erste Schritt die Umwandlung des Ethanols in Acetaldehyd in Gegenwart eines Katalysators ermöglicht, der aus einem Gemisch aus Chromoxid und Kupferoxid oder jedem anderen geeigneten Katalysator besteht, wobei das Massenverhältnis von Ethanol zu Acetaldehyd in dem Abstrom aus dem ersten Reaktionsschritt vorzugsweise zwischen 2:1 und 4:1 liegt, wobei der ethanolreiche Rohstoff und der aus dem Trennschritt B stammende Ethanolabstrom, gemischt mit dem Abstrom aus dem ersten Reaktionsschritt, in den zweiten Reaktionsschritt eingespeist werden.

6. Verfahren nach Anspruch 5, wobei der zweite Reaktionsschritt von Schritt A in Gegenwart eines Katalysators vom Typ Siliciumdioxid mit einem Tantal-, Zirkon oder Nioboxid, durchgeführt wird, wobei der zweite Reaktionsschritt von Schritt A bei einer Temperatur im Bereich zwischen 320 und 370 °C durchgeführt wird, wobei der erste Reaktionsschritt von Schritt A bei einer Temperatur im Bereich zwischen 200 und 300 °C durchgeführt wird.

7. Verfahren nach einem der Ansprüche 1 bis 6, wobei der Trennschritt B ausgewählt ist aus den Schritten Destillation, Tieftemperaturdestillation, Waschen mit Lösungsmittel, Extraktivdestillation, Flüssig-Flüssig-Extraktion, Durchleiten durch ein Sieb, Membrantrennung und Kombinationen dieser Schritte.

8. Verfahren nach einem der Ansprüche 1 bis 7, wobei der Schritt C eine indirekte Hydrierung ist, wobei in einem ersten Reaktionsschritt Ethylen in Gegenwart von konzentrierter Schwefelsäure bei einer Reaktionstemperatur im Bereich zwischen 50 und 150 °C in einem zweiphasigen gasförmigen/flüssigen Medium umgesetzt wird, und in einem zweiten Schritt die in dem ersten Schritt gebildeten Produkte hydrolysiert werden, um bei einer Temperatur im Bereich zwischen 70 und 100 °C überwiegend Ethanol zu bilden.

9. Verfahren nach einem der Ansprüche 1 bis 7, wobei Schritt C eine direkte Hydrierung ist, die in einer Gasphase bei einer Reaktionstemperatur im Bereich zwischen 200 und 400 °C in Gegenwart eines heterogenen Katalysators auf der Basis von anorganischen Säuren, wie Phosphorsäure, abgeschieden auf aus Siliciumdioxid-basierten Trägern, durchgeführt wird.

10. Verfahren nach einem der Ansprüche 1 bis 9, wobei der ethanolreiche Rohstoff Acetaldehyd umfasst, wobei das Massenverhältnis von Ethanol zu Acetaldehyd zwischen 2:1 und 4:1 liegt.

11. Verfahren nach einem der Ansprüche 1 bis 10, wobei die Reinheit des Ethylenabstroms, der in den Hydrierungsschritt C eingespeist wird, zwischen 65 und 99,9 Gew.-% liegt.

12. Verfahren nach einem der Ansprüche 1 bis 10, wobei die Reinheit des Ethylenabstroms, der in den Hydrierungsschritt C eingespeist wird, zwischen 65 und 75 Gew.-% liegt.

## Claims

1. Process for the production of 1,3-butadiene from a feedstock rich in ethanol, i.e. in which the ethanol represents more than 50% of the total weight of said feedstock, comprising at least:
A) A stage of conversion of at least said feedstock rich in ethanol and of the ethanol effluent obtained from separation stage B to a conversion effluent comprising a majority of 1,3-butadiene, water and ethylene, and to a hydrogen effluent, operating at a pressure comprised between 0.1 and 1.0 MPa, at a temperature comprised between 300 and 500°C in the presence of at least one catalyst;
B) A stage of separation of at least said conversion effluent obtained from A and the hydration effluent obtained from C to at least an ethanol effluent, a butadiene effluent, a water effluent and an ethylene effluent;
C) A stage of hydration of the ethylene fed at least by said ethylene effluent and/or said water effluent both obtained from separation stage B, in order to produce a hydration effluent comprising ethanol, said hydration effluent then being recycled to the separation stage B.

2. Process according to claim 1, wherein said stage **A** is operated at a pressure comprised between 0.1 and 0.5 MPa.

3. Process according to one of claims 1 to 2, wherein said conversion stage **A** is operated in one reaction stage and wherein said feedstock rich in ethanol is mixed with the ethanol effluent obtained from the separation stage **B** before feeding said conversion stage **A.**

4. Process according to claim 3, wherein said stage **A** is operated in the presence of a catalyst of zinc aluminate type or chromium-doped MgO-SiO₂ type at a temperature comprised between 380 and 430°C.

5. Process according to one of claims 1 to 2, wherein said stage **A** is operated in two reaction stages, the first stage making it possible to convert the ethanol to acetaldehyde in the presence of a catalyst consisting of a mixture of chromium oxide and copper oxide, or of any other suitable catalyst, the mass ratio of ethanol to acetaldehyde in the effluent of said first reaction stage being preferentially comprised between 2:1 and 4:1, wherein said feedstock rich in ethanol feeds said first reaction stage and said ethanol effluent obtained from said separation stage **B** feeds said second reaction stage, in a mixture with said effluent from said first reaction stage.

6. Process according to claim 5, wherein the second reaction stage of said stage **A** is operated in the presence of a catalyst of silica type with an oxide of tantalum, zirconium or niobium, wherein the second reaction stage of stage **A** is operated at a temperature comprised between 320 and 370°C, the first reaction stage of said stage **A** is operated at a temperature comprised between 200 and 300°C.

7. Process according to one of claims 1 to 6, wherein the separation stage **B** is chosen from the stages of: distillation, cryogenic distillation, washing with solvent, extractive distillation, liquid-liquid extraction, passing through a sieve, membrane separation and the combinations of these stages.

8. Process according to one of claims 1 to 7, wherein said stage **C** is an indirect hydration, wherein, in a first reaction stage, the ethylene reacts in the presence of concentrated sulphuric acid at a reaction temperature comprised between 50 and 150°C, in a two-phase gas/liquid medium, and in a second stage, the products formed in the first stage are hydrolyzed in order to form a majority of ethanol at a temperature comprised between 70 and 100°C.

9. Process according to one of claims 1 to 7, wherein said stage **C** is a direct hydration operating in gas phase, at a reaction temperature comprised between 200 and 400°C, in the presence of a heterogeneous catalyst based on inorganic acids such as phosphoric acid deposited on supports based on silica.

10. Process according to one of claims 1 to 9, wherein said feedstock rich in ethanol comprises acetaldehyde, the mass ratio of ethanol to acetaldehyde being comprised between 2:1 and 4:1.

11. Process according to one of claims 1 to 10, wherein the purity of the ethylene flow that feeds hydration stage **C** is comprised between 65 and 99.9% by weight.

12. Process according to one of claims 1 to 10, wherein the purity of the ethylene flow that feeds hydration stage **C** is comprised between 65 and 75% by weight.
